(19) **Europäisches Patentamt**

**European Patent Office**

**Office européen des brevets**

(11) **EP 0 660 734 B1**

(12) **EUROPÄISCHE PATENTSCHRIFT**

(45) Veröffentlichungstag und Bekanntmachung des Hinweises auf die Patenterteilung:
**17.12.1997 Patentblatt 1997/51**

(21) Anmeldenummer: **93919016.1**

(22) Anmeldetag: **16.09.1993**

(51) Int. Cl.$^6$: **A61N 1/05**, A61N 1/37

(86) Internationale Anmeldenummer:
**PCT/DE93/00887**

(87) Internationale Veröffentlichungsnummer:
**WO 94/06508 (31.03.1994 Gazette 1994/08)**

(54) **HERZSCHRITTMACHERSYSTEM**

PACEMAKER SYSTEM

SYSTEME DE STIMULATION CARDIAQUE

(84) Benannte Vertragsstaaten:
**DE FR NL**

(30) Priorität: **17.09.1992 DE 4231603**

(43) Veröffentlichungstag der Anmeldung:
**05.07.1995 Patentblatt 1995/27**

(73) Patentinhaber:
**BIOTRONIK Mess- und
Therapiegeräte GmbH & Co
Ingenieurbüro Berlin
D-12359 Berlin (DE)**

(72) Erfinder:
• **BOLZ, Armin
D-91052 Erlangen (DE)**

• **SCHALDACH, Max
D-91054 Erlangen (DE)**

(74) Vertreter:
**Christiansen, Henning, Dipl.-Ing. et al
Patentanwalt
Pacelliallee 43/45
14195 Berlin (DE)**

(56) Entgegenhaltungen:
**DE-A- 3 501 169**

• **BIOMEDIZINISCHE TECHNIK, Bd.34, Nr.7/8,
August 1989, BERLIN, DE pages 185 - 190
SCHALDACH 'Titannidrid-Herzschrittmacher-
Elektroden'**

## Beschreibung

Die Erfindung betrifft ein Herzschrittmachersystem der im Oberbegriff des Anspruchs 1 angegebenen Art.

Ein solches ist aus DE-A-3 501 169 bekannt.

Bei der Entwicklung von künstlichen Herzschrittmachern wird seit langem angestrebt, den Erfolg einer Stimulation des Herzens durch die Messung und Auswertung von Signalen zu überprüfen, welche aufgrund der evozierten Herzaktion über eine im Herzen verlegte Elektrode - und vorzugsweise die Stimulationselektrode selbst - im Herzen aufnehmbar sind.

Die Aufnahme des elektrischen "Antwortsignals" des Herzens nach einer Stimulation ist durch die Nachwirkungen des Stimulationsimpulses gestört, welche ihre Ursache in der Polarisation des stimulierten Gewebes haben, die erst abgebaut werden kann, wenn auch die mit dem Stimulationsimpuls verbundene Umladung des Ausgangs(koppel)kondensators beseitigt ist.

Dies folgt daraus, daß das den Erfolg der Stimulation anzeigende und am Herzen etwa bis 300 ms nach der Stimulation vorhandene evozierte Potential von einem Nachpotential in der Größenordnung von mehr als 10 mV überlagert wird. Das die Effektivitätserkennung störende Nachpotential entsteht durch die Wirkung der Stimulationselektrode als elektrochemische Elektrode, woraus eine Übersteuerung des Detektionsverstärkers resultiert.

Neben verschiedenen Schaltungen, mit denen versucht wird, die Folgen des Nachpotentials zu beseitigen, ist aus der EP-B1-0 000 989 eine Vorrichtung zur Herzstimulation bekannt, bei der das störende Nachpotential der Stimulationselektrode durch einen zusätzlichen, transistorgesteuerten und die Helmholtz-Kapazität im wesentlichen kurzschließenden Widerstandszweig beschleunigt abgebaut werden soll. Die vollständige zum Abbau des störenden Nachpotentials notwendige Zeit ist bei üblichen Elektroden jedoch zu lang, um eine unter allen Umständen wirksame Effektivitätserkennung zu ermöglichen.

Aus M. Schaldach et al.: "Titannitrid-Herzschrittmacher-Elektroden", Biomedizinische Technik 34 (1989), Nr. 7/8, S. 185, sind poröse TiN-Elektroden mit verbesserten Eigenschaften bekannt.

Ausgehend von den Mängeln des Standes der Technik liegt der Erfindung die Aufgabe zugrunde, ein Herzschrittmachersystem der eingangs genannten Gattung anzugeben, bei dem eine wirksame Erkennung von evozierten Herzsignalen auch unter den verschiedenen sich verändernden Betriebsbedingungen eines Herzschrittmachers, die sich beispielsweise beim Einheilen der Elektrode ergeben, wirksam möglich ist.

Diese Aufgabe wird mit den kennzeichnenden Merkmalen des Anspruchs 1 gelöst.

Die Erfindung schließt die Erkenntnis ein, daß sich beim Anlegen einer elektrischen Spannung an eine im Herzen verankerte Schrittmacherelektrode zwei Schichten unterschiedlicher Ladungsträger ausbilden die jedoch aufgrund von Hydratisierungseffekten durch eine Monolage von Wasserstoffmolekülen getrennt sind. Diese sogenannte Helmholtzsche Doppelschicht entspricht im Aufbau und in ihrem elektrischen Verhalten einem Plattenkondensator. Fließt bei der Stimulierung des Herzens ein Strom über diese Helmholtz-Kapazität, so entsteht dort eine das Nachpotential bildende Spannung, die mit abnehmender Helmholtz-Kapazität zunimmt. Durch weitere, an der Phasengrenze stattfindende elektrochemische Reaktionen mit geladenen Reaktionsprodukten wird das Nachpotential zusätzlich erhöht. Neben der Erhöhung der Helmholtz-Kapazität ist vor allem eine Verringerung des Stimulationsamplitude für das Absenken des Nachpotentials von Bedeutung, um eine eindeutige Effektivitätserkennung mit einer Einzelelektrode durchführen zu können. Eine Reduzierung der Amplitude der Stimulationsimpulse trägt darüberhinaus in vorteilhafter Weise zur Erhöhung der Lebensdauer der Stromversorgungsquelle des Schrittmachers bei.

Durch die Wahl die erfindungsgemäßen Maßnahmen läßt sich also einerseits die Stimulationsamplitude verringern, so daß das Nachpotential insgesamt geringer wird. Ferner wird der Abbau des Nachpotentials beschleunigt, so daß es innerhalb eines vorgegebenen Zeitraums definiert abbaubar ist.

Dieser Abbau kann durch einen aktiven Gegenimpuls oder auch durch passive Mittel am Ausgang der Stimulationsschaltung erfolgen, wie es aus dem genannten Stand der Technik bekannt ist (Autoshort).

Gemäß vorteilhafter Weiterbildungen der Erfindung wird durch selbsttätig arbeitende Schaltungsmittel die Möglichkeit geschaffen, die Zeitdauer der Sperrung des Eingangsverstärkers für die evozierten Impulse selbsttätig zu ermitteln und an die Implantationsbedingungen bzw. deren zeitliche Veränderung anzupassen. Hierbei wird eine erhöhte Reizenergie verwendet, welche mit Sicherheit zu einer Stimulation führt.

Ferner läßt sich in vorteilhafter Weiterbildung ein Herzschrittmachersystem angeben, welches durch selbsttätige Anpassung auch der Stimulationsamplitude insgesamt nur einen niedrigen Energiebedarf hat.

Dabei wurde erkannt, daß

- ein Stimulierungsimpuls mit überhöhter Amplitude zwar mit Sicherheit zu einer Reizung des Myocards führt, aber aufgrund des erhöhten Energieverbrauchs die Lebensdauer der Stromversorgungsquelle des Schrittmachers erheblich reduziert, so daß eine vorzeitige Reimplantation vorgenommen werden muß,

- eine ausreichend sichere Detektierung von evozierten Signalen aufgrund einer erfolgten Myocard-Reizung erst nach ausreichendem Abbau des sich durch den Stimulierungsimpuls einstellenden Nachpotentials möglich ist, wenn Stimulierung und Detektierung mit der gleichen Elektrode durchgeführt wird,

- das Nachpotential höchstens einen solchen Wert annehmen darf, der in einem Zeitraums von etwa 30 bis 80 ms bis auf ein vernachlässigbares Niveau abgebaut werden kann (Autoshort), bevor ein evoziertes Potential abgeklungen ist
und

- die Werkstoffe der bekannten Elektroden und insbesondere Titan, Vanadium, Zirkon und Niob zu teilweise extremer Oxidation neigen und daß diese hohe Oxidationsneigung bei Kontakt zu wässrigen Elektrolyten dazu führt, daß sich an der Elektrodenoberfläche eine dünne, isolierende bzw. halbleitende Oxidschicht bildet, die eine der Helmholtzkapazität $C_H$ in Serie geschaltete Kapazität $C_{ox}$ darstellt und so zur langsamen Verringerung der Gesamtkapazität und damit zur entsprechenden Erhöhung der jeweils erforderlichen Stimulationsenergie führt.

Die Impulssteuerung des erfindungsgemäßen Herzschrittmacher-Systems ist sowohl für die selbsttätige Ermittlung der, für die Detektion evozierter Potentiale notwendigen Breite der Autoshort-Impulse als auch für die Einhaltung einer minimalen, über der Reizschwelle des Myocard liegenden Amplitude der Stimulationsimpulse bei der ermittelten notwendigen Breite der Autoshort-Impulse ausgebildet und weist die dazu erforderlichen elektrischen Mittel auf. Dazu gehören im wesentlichen ein regelbarer Autoshort-Impulsgenerator, ein Generator zur Erzeugung von, durch eine Torschaltung gesteuert, amplitudengeregelten Stimulationsimpulsen bei vorgegebener Impulsfolgefrequenz und Einrichtungen zur Detektion der durch diese Impulse evozierten Potentiale in Abhängigkeit von der Breite der Autoshort-Impulse. Die Selbsteinstellung der Autoshort-Zeit gehört zu den wesentlichsten Vorteilen der Impulssteuerung.

Der Betrieb der hier dargestellten Impulssteuerschaltung erfolgt in zwei unterschiedlichen Betriebszuständen "Abgleich" und "Dauerbetrieb". Nach der bevorzugten Ausführungsform der Erfindung ist für die Erzeugung der Autoshort-Impulse ein Impulsgenerator vorgesehen, bei dem eine Variation der Impulsbreite im Betriebszustand "Abgleich" in scannender Weise durch einen Rampengenerator vorgenommen wird. Die Stimulationsimpulse sind hierbei durch eine entsprechende Einstellung in der Impulsamplitudensteuerung des Stimulationsimpuls-Generators in ihrer Amplitude in einer Höhe konstant gehalten, welche über der Reizschwelle des Myocards liegt und bei der mit Sicherheit ein evoziertes Potential ausgelöst wird. Die entsprechend detektierten, impulsförmigen Signale werden nach ausreichender Verstärkung einem Speicher zugeführt.

Der Speicher ist matrix- oder array-artig ausgebildet und wird von dem zuvor genannten Rampengenerator derart adressiert, daß eine Zuordnung der Speicherplätze zu den evozierten Signalen in Abhängigkeit von den jeweiligen Autoshort-Impulsen bestimmter Breite erfolgt.

Eine dem Matrix-Speicher nachgeordnete Auswerte-Einheit ermittelt die effektivste Detektierung der evozierten Potentiale in Bezug auf die Impulsbreite der Autoshort-Impulse. Diese Autoshort-Zeit wird in dem Generator für die Autoshort-Impulse fixiert und legt als selbsteingestellter Wert die Breite der Autoshort-Impulse für den dem Betriebszustand "Abgleich" folgenden "Dauerbetrieb" der Impulssteuerung fest. Für die Umschaltung der Betriebszustände ist ein zyklischer Zeitschalter vorgesehen, durch den der Rampengenerator, der Generator für die Autoshort-Impulse und die Amplitudensteuerungsstufe der Stimulationsimpulse entsprechend zu- oder abschaltbar sind. Während des "Dauerbetriebs" der Impulssteuerung wird eine am Eingang der Amplitudensteuerungsstufe für die Stimulationsimpulse vorgesehene Torschaltung durch den Zeitschalter und die Detektionsimpulse der evozierten Potentiale aktiviert.

Jeder einem detektierten Potential entsprechende Impuls führt zu einer Reduzierung der Amplitude der Stimulationsimpulse um einen bestimmten Betrag. Unterschreitet der Stimulationsimpuls nach einer Anzahl von Stimulationen die Reizschwelle, so kann kein evoziertes Potential mehr aufgenommen werden. Ein entsprechendes Ausgangssignal an einer Torschaltung führt in der nachgeordneten Amplitudensteuerungsstufe zu einer Erhöhung der Amplitude der Stimulationsimpulse auf den zuletzt erfolgreich angewandten Wert. Dadurch wird erreicht, daß der nach fehlender Detektion eines evozierten Potentials folgende Stimatiionsimpuls mit Sicherheit zu einer erneuten Reizung des Myocards führt und ein "Außer-Tritt-Kommen" der Synchronisation des Gesamtsystems vermieden wird.

Es ist ersichtlich, daß anstelle des der Stimulationsamplitude auch die Impulsbreite oder ein anderer die Reizenergie bestimmender Wert verändert werden kann.

Besonders günstig ist es auch, wenn die Kompensation des Nachpotentials durch einen aktiven Gegenimpuls erfolgt, da die bei dem erfindungsgemäßen Herzschrittmacher-System verwendete Elektrode auch anodisch betrieben werden kann, ohne daß eine Oxidschicht die Stimulationsschwelle beeinträchtigt.

Entsprechend einer günstigen Weiterbildung der Erfindung ist die Amplitudenerhöhung bei fehlender Detektions eines evozierten Potentials mehrfach so groß, wie der Wert der Amplitudenabsenkung bei erfolgter Detektion. Dies wird auf einfache Weise durch eine Teiler-Schaltung erreicht, die die Ausgangssignale der Torschaltung zur Amplitudenreduzierung mit diesem Faktor versieht.

Gemäß einer Ausführungsform der Erfindung erfolgt ein Wechsel der Schaltzustände des Zeitschalters in sich zyklisch wiederholenden Zeitabschnitten gleicher Länge, um regelmäßig eine Kontrolle der gewählten Autoshort-Zeit vorzunehmen. Es hat sich als günstig erwiesen, nach einer vorgegebenen Anzahl von Absenkzyklen der Amplitude der Stimulationsimpulse bis zum ersten Ausbleiben einer Potential-Detektion erneut einen "Abgleich" durchzuführen, um die Autoshort-Zeit gegebenenfalls an eine mögliche Veränderung der Reizfähigkeit des Myocards selbstständig anzupassen.

Die Funktion der erfindungsgemäßen Impulssteuerung ist für Autoshort-Zeiten im Bereich von 50 ms nur gewährleistet, wenn durch die konstruktive Gestaltung der Stimulationselektrode erreicht wird, daß sich nur ein relativ niedriges Nachpotential nach dem Stimulationsimpuls aufbaut.

Nach der Erfindung besitzt die Stimulationselektrode eine poröse Oberflächenbeschichtung aus einem inerten Material, deren aktive Oberfläche wesentlich größer ist, als die sich aus der geometrischen Form der Elektrode ergebende Oberfläche. Wegen der bevorzugt fraktalen räumlichen Geometrie ist die aktive Oberfläche so groß, daß die zur Stimulation erforderliche Energie auf einen minimalen Wert einstellbar ist. Damit ist den herkömmlichen beschichteten porösen Elektroden wegen ihrer großen relativen Oberfläche zunächst grundsätzlich eine Stimulation mit gutem Erfolg bei niedriger Energie möglich. Es wurde nun erkannt, daß durch die Oxidationsneigung die Helmholtzkapazität verkleinert wird, was zu einer Erhöhung der Elektrodenimpedanz führt. Die damit hervorgerufene Beeinflussung der Elektrodeneigenschaften im Laufe der Implantationszeit ist deshalb so schwerwiegend, weil die Verschlechterung der Elektrodeneigenschaften Auswirkungen hat, welche ihrerseits dazu beitragen, daß die Stimulationseigenschaften zusätzlich ungünstig beeinflußt werden.

So ist bei einer sich verschlechternden Elektrode eine höhere Impulsenergie notwendig, so daß zur Effektivitätserkennung auch ein Gegenimpuls mit größerer Energie erforderlich ist, der seinerseits wieder zur Verschlechterung der Elektrodeneigenschaften beiträgt. Da die Impulsenergie und die zur Effektivitätserkennung notwendigen Gegenimpulse auf Werte eingestellt sind, welche über die gesamte Implantationsdauer des Schrittmachers Gültigkeit haben müssen, beruht die Verschlechterung der Betriebsbedingungen, im Endeffekt im wesentlichen auf Maßnahmen, welche den verschlechterten Betbedingungen eigentlich entgegenwirken sollen.

Die langzeitstabile, bioverträgliche Oberflächenbeschichtung der erfindungsgemäßen Stimulationselektrode besteht aus einem Material, dessen Oxidationsneigung sehr gering ist, wobei sie vorzugsweise unter Verwendung eines inerten Materials, also eines Nitrides, Carbides, Carbonitrides oder aber eines reinen Elementes bzw. bestimmter Legierungen aus der Gruppe Gold, Silber, Platin, Iridium, Titan oder Kohlenstoff vakuumtechnisch auf die Elektrode aufgetragen wird. Wegen der fraktalen räumlichen Geometrie einer derart aufgetragenen Oberflächenschicht ist deren aktive Oberfläche sehr groß, so daß die zur Stimulation erforderliche Energiemenge äußerst gering gehalten werden kann.

Das Nachpotential einer Stimulationselektrode aus Titan, die mittels der reaktiven Kathodenzerstäubung eine gesputterte Iriduimnitrid- oder Titannitridschicht aufweist, ist bis um das sechsfache (von ca. 600 auf ca. 100 mV) kleiner als das Nachpotential einer blanken Stimulationselektrode aus Titan. Wegen dieser signifikanten Verringerung des Nachpotentials ist die Erkennung des intrakardialen EKGs nicht nur auf herkömmliche Weise mit einem Verstärker und einer Triggereinrichtung möglich, sondern es kann eine funktionsfähige Effektivitätserkennung angewandt werden, die ohne Gegenimpuls und Autoshort-Zeiten zum Abbau des Nachpotentials in der Größenordnung von 50 ms auskommt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Blockschaltbild der bevorzugten Ausführungsform der Erfindung,

Figur 2 ein Übersichtsdiagramm mit den wichtigsten Einflußgrößen für die Impulssteuerung in schematisierter Darstellung,

Figur 3 ein in schematisierter Form dargestelltes Amplituden-Zeit-Diagramm für die erzeugten Stimulationsimpulse und detektierten Evotionspotentiale,

Figur 4 ein Ausführungsbeispiel einer Stimulationselektrode in schematischer Darstellung in Seitenansicht,

Figur 5 eine vergrößerte Darstellung des Detail A in Figur 4 im Schnitt,

Figur 6 ein Diagramm zum Vergleich der Impedanz des Ausführungsbeispiels der Elektrode mit der Impedanz von aus dem Stand der Technik bekannten entsprechenden Elektroden gleicher geometrischer Abmessungen sowie

Figur 7 eine Darstellung des Nachpotentials als Funktion der Autoshort-Zeit in Abhängigkeit von der Oberflächengestaltung der Elektrode.

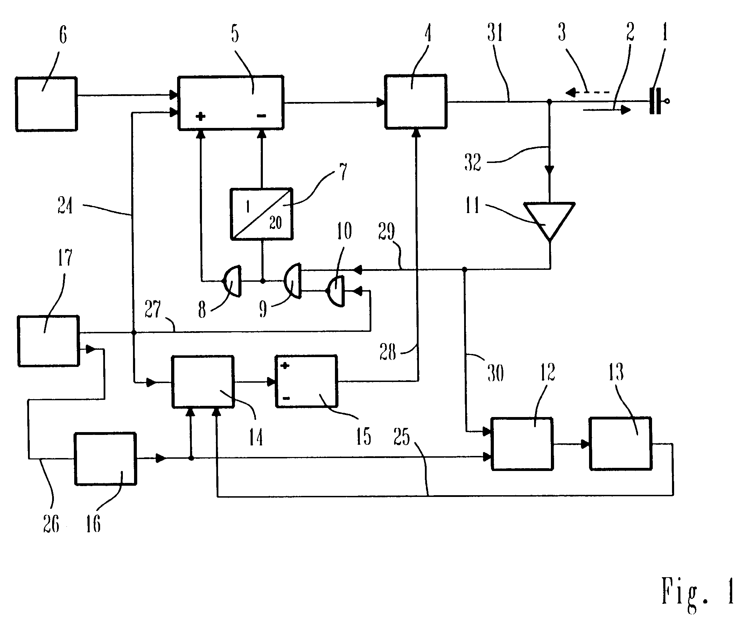

Die Mittel zur Impulserzeugung des erfindungsgemäßen Herzschrittmacher-Systems ist in Figur 1 schematisch dargestellt. Die zu dem Herzschrittmacher-Systems gehörende Stimulationselektrode ist an den Ausgangskondensator 1 angeschlossen und in der Zeichnung nicht dargestellt.

Das System weist einen Impulsgenerator 4 für Stimulationsimpulse auf, welche in der Richtung 2 auf die Stimulationselektrode abgegeben werden.

Eine Zeitsteuerstufe 6 bestimmt den Zeitpunkt der Abgabe von Stimulationsimpulsen und entspricht in diesem Fall einem starrfrequenten Schrittmacher, wobei das dargestellte Prinzipschaltbild auch für andere Schrittmacherschaltungen verwendet werden kann, wobei lediglich zusätzliche Steuerleitungen vorzusehen sind, durch die beispielsweise bei einem Demand-Schrittmacher die Abgabe von Stimulationsimpulsen bei vor dem Ende des sogenannten Escape-Intervalle eingehenden von Herzaktionen herrührenden Signalen eine Stimulation unterbunden wird. Mit einer Amplitudensteuerstufe 5 läßt sich die Amplitude (bzw. die Energie) der Stimulationsimpulse über zusätzliche Eingänge herauf- ("+") bzw. herunter- ("-") setzen.

Ferner ist ein Impulsgenerator 15 zur Erzeugung von Autoshort-Impulsen über die Endstufe 4 vorgesehen. Über ein galvanisches Verbinden oder einen aktiven Gegenimpuls wird dabei das Potential des inneren Anschlusses des Kondensators 1 in den Ausgangszustand vor dem letzten Stimulationsimpuls zurückgeführt, so daß durch die erzeugte Ladungsverschiebung dem Nachpotential an der Elektrode entgegengewirkt wird. Die Zeitdauer des Impulses zur Beseitigung der Nachwirkungen des Stimulationsimpulses ist über einen entsprechenden Eingang des Impulsgenerators 15 einstellbar.

Über einen Verstärker 11 werden vom Herzen hervorgerufene Signale aufgenommen, wobei der Verstärker durch nicht dargestellte Schaltungsmittel beim Auftreten eines Stimulationsimpulses unempfindlich geschaltet wird. In einem Speicher 12 wird ein evoziertes Ereignis festgehalten. Um die Zeitdauer des Autoshort-Impulses optimieren zu können, wird ein ein evoziertes Ereignis anzeigendes Signal in Zuordnung zur Dauer des entsprechenden Autoshort-Impulses festgehalten.

Mittels eines Steuerschalters 17 erfolgt die Einstellung der Betriebsart "Abgleich", während die Schaltung sich im übrigen in der Betriebsart "Dauerbetrieb" befindet.

Im getriebszustand "Abgleich" erfolgt die Ermittlung der optimalen Autoshort-Zeit, die dann in der Stellung "Dauerbetrieb" beibehalten wird. Dazu wird durch den Zeitschalter 17 über die Steuerleitung 24 in der Amplitudensteuerung 5 bei konstanter Impulsfolgefrequenz (Zeitsteuerung 6) eine Impulsamplitude vorgegeben, bei der mit Sicherheit ein evoziertes Potential am Myocard entsteht. Gleichzeitig aktiviert der Zeitschalter 17 über die Steuerleitung 26 einen Rampengenerator 16, der über einen Umschalter 14 an den Impulsgenerator 15 angeschlossen ist, um die Breite der Autoshort-Impulse scannend zu variieren. Ebenfalls durch den Zeitschalter 17 über die Leitung 27 und einen Negator 10 gesteuert, wird das UND-Gatter 9 gesperrt.

Ein aufgenommenes evoziertes Potential oder ein entsprechendes, diesen Zustand anzeigendes Signal 3 wird über die Verbindungsleitung 32 einem Verstärker 11 zugeführt und in einem Matrix-Speicher 12 erfaßt. Die Zuordnung der einzelnen Speicherplätze erfolgt in Abhängigkeit der Zeitfunktion des Rampengenerators 16, so daß jeder Impulsbreite ein Signal zugeordnet werden kann, welches die Aufnahme eines evozierten Potentials anzeigt. Durch eine Auswerteschaltung 13 wird die zur Detektion der evozierten Potentiale 3 günstigste Autoshort-Zeit ermittelt. Hierbei wird ein Mittelwert aller Impulsdauern des Autoshort-Impulses ausgewählt, bei denen ein evoziertes Signal aufgenommen werden konnte, um eine gewisse Sicherheit gegenüber der Veränderung von Signalaufnahmebedingungen im Verlaufe der Betriebszeit des Schrittmachers zu haben.

Anschließend erfolgt ein Zurücksetzen des Schalters 17 auf den Betriebszustand "Dauerbetrieb", wobei der mittlere Wert der Autoshort-Zeit, bei der ein evoziertes Potential aufgenommen werden konnte, über den Umschalter 14 und die Leitung 25 im Impuls-Generator 15 festgehalten und das UND-Gatter 9 über den Negator 10 freigeschaltet wird. Anschließend wird die Stimulationsamplitude wieder auf ihren Normalwert abgesenkt.

Nunmehr ist es möglich, mit den durch den vorgenommenen Abgleich sicher erkennbaren evozierten Potentialen bei dem Betrieb mit Schwellwertsteuerung über eine Effektivitätserkennung die Stimulationsenergie (-amplitude) so einzustellen, daß die Stimulationsschwelle sicher überschritten ist, ohne daß durch eine überhöhte Stimulationsenergie eine vorzeitige Erschöpfung der Energiequelle erfolgt.

Jede Detektion eines evozierten Potentials 3 erzeugt über das UND-Glied 9 am Teiler 7 einen Impuls, der die Amplitude des nächsten Stimulationsimpulses 2 um einen bestimmten Betrag verringert. Diese schrittweise Amplitudenabsenkung erfolgt solange, bis bei der vorgegebenen Autoshort-Zeit kein evoziertes Potential detektiert wird. Die Pegeländerung am Ausgang des UND-Gatters 9 schaltet den Negator 8 und bewirkt daraufhin ein Anheben der Stimulationsamplitude bis zu einem vorangehenden Wert, bei dem eine Stimulation sicher erfolgte. Durch den Teiler 7 erfolgt eine Amplitudenabsenkung nur bei jedem n-ten (hier 20.; dieser Wert stellt nur ein Beispiel dar, da sich in Praxis die Reizschwelle langfristig stabilisieren wird, so daß Teilerverhältnisse von einigen Tausend praktikabel sein werden) erfolgreichen Stimulationsimpuls - ein Anheben jedoch unmittelbar nach jeder fehlgeschlagenen Stimulation. Damit weisen die Stimulationsimpulse stets eine nur geringfügig über der Reizschwelle liegende Reizenergie, insbesondere Amplitude auf, welche jeweils mit großer Sicherheit zu einer Herzstimulation führt.

Um mögliche Veränderungen der Übertragungsverhältnisse des Myocards zu erfassen, ist es von besonderem Vorteil, nach einer "Dauerbetrieb"-Phase der Impulssteuerung in einer Wiederholung eines "Abgleichs" erneut die für die Stimulierung und Detektierung der Herzaktivität optimale Autoshort-Zeit zu bestimmen. Es hat sich als günstig erwiesen, nach einem "Dauerbetrieb" mit beispielsweise m Zyklen für die Amplitude der Stimulierungsimpulse einen weiteren "Abgleich" durchzuführen. Darüberhinaus ist es möglich, den Umschaltzyklus des Schalters 17 den patienten spezifischen Bedingungen anzupassen.

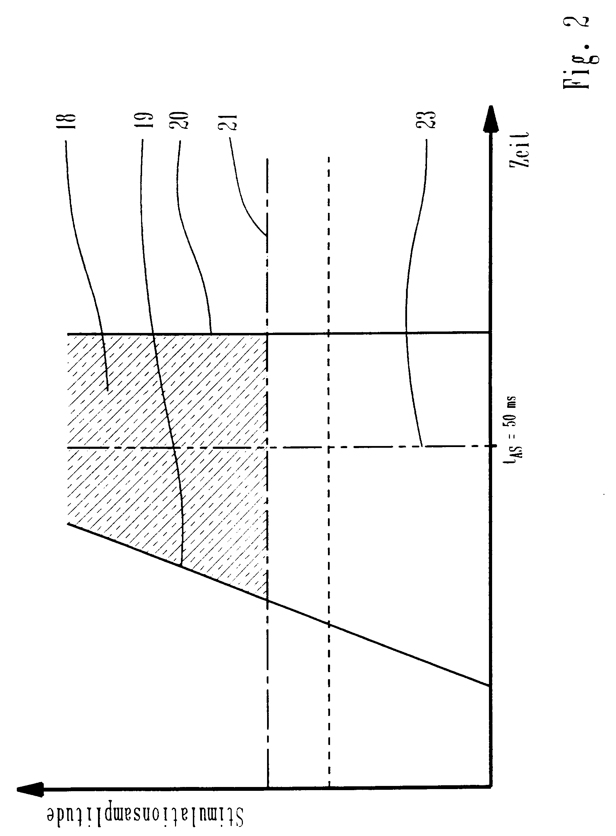

Das in Figur 2 schematisiert dargestellte Diagramm zeigt auf der Zeitachse die Möglichkeit der Aufnahme evozierter Potentiale im Herzen in Abhängigkeit von der Variation der Autoshort-Zeit und der Stimulationsamplitude.

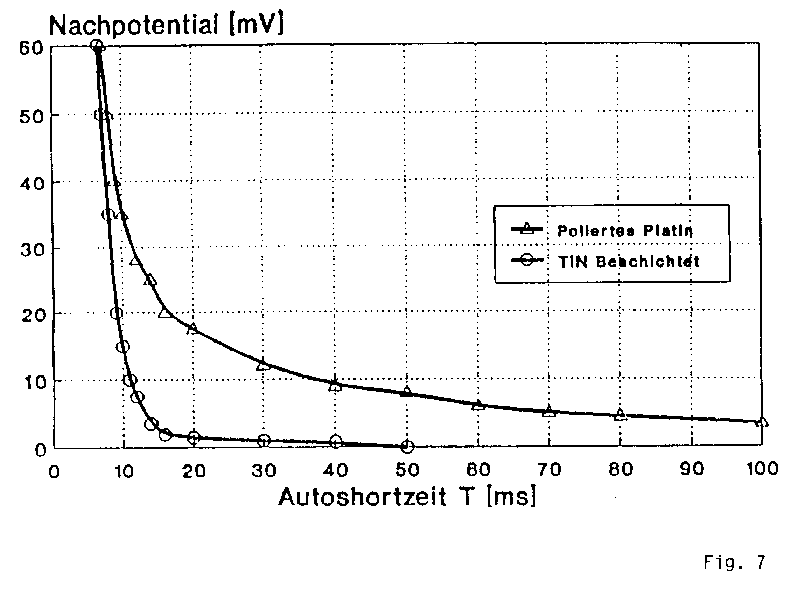

Evozierte Signale können nur aufgenommen werden, wenn ein Stimulationsimpuls wirksam ist, er also eine vorgegebene Schwellenenergie überschritten hat, wie sie durch die horizontale Linie 21 angegeben ist. Ferner wird die Möglichkeit der Aufnahme evozierter Signale noch begrenzt durch das Abklingen des evozierten Potentials, was durch Linie 19 als Grenze für das Abklingen der Stimulationswirkung (Nachpotential) angedeutet ist. Die Linie ist leicht geneigt, da mit erhöhter Stimulationsamplitude auch das (störende) Nachpotential ansteigt bzw. die Zeitdauer von dessen Abklingen größer wird. Der Zeitpunkt 20 bildet diejenige Zeitmarke, nach welcher ein evoziertes Potential auf einen so geringen Wert abgeklungen ist, daß dessen Detektierung nicht mehr möglich bzw. das interessierende Ereignis vorbei ist. Mit den erfindungsgemäßen Maßnahmen wird bei einer Selbsteinstellung der Dauer der Autoshort-zeit ein Zeitbereich für die Maßnahmen zur Beseitigung des Nachpotentials eingestellt, der in dem effektiven Bereich liegt. Zwischen den durch die Linien 19 und 20 entstehenden Grenzwerten wird insbesondere ein mittlerer Wert eingestellt. Die erfindungsgemäße Beschichtung der Stimulationselektrode ermöglicht ein Absenken des die Detektion der evozierten Potentiale störenden Nachpotentials bei einer Autoshort-Zeit von 50 ms auf einen Wert von nahezu 0 mV (vergleiche Figur 7).

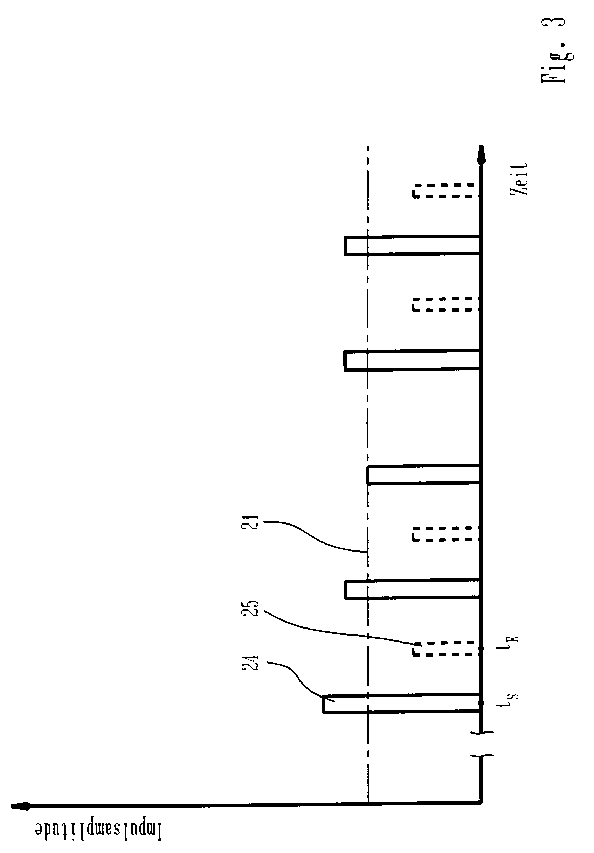

Figur 3 zeigt das Amplituden-Zeit-Diagramm der Stimulationsimpulse 24 in Relation zu detektierbaren evozierten Potentialen 25 während des Betriebszustands "Dauerbetrieb" der Impulssteuerung. Nach jedem Stimulationsimpuls 24, für den ein evoziertes Potential 25 nach der Autoshort-Zeit $T = t_E - t_S$ detektiert wird, erfolgt über die Impulsamplitudensteuerung (vergleiche Position 5 in Figur 1) eine schrittweise Amplitudenabsenkung. Wird die Detektionsgrenze mit der Reizschwelle erreicht oder geringfügig unterschritten, so bewirkt die resultierende Potentialänderung am Ausgang der Torschaltung (vergleiche die Positionen 7, 8, 9, 10 in Figur 1) ein erneutes Ansteigen der Amplitude des nachfolgenden Stimulationsimpulses 24. Der Amplitudensprung erfolgt insbesondere auf den Amplitudenwert, bei dem zuletzt eine erfolgreiche Stimulation erfolgte.

Um die Zahl der Reizschwellenunterschreitungen, bei denen keine effektive Stimulation erfolgt, möglichst gering zu halten, wird bei vorteilhaften Ausführungen der Erfindung nur eine Absenkung bei jedem n-ten Stimulationsimpuls vorgenommen, wobei ein Anheben unmittelbar nach jeder Schwellenunterschreitung vorgenommen wird.

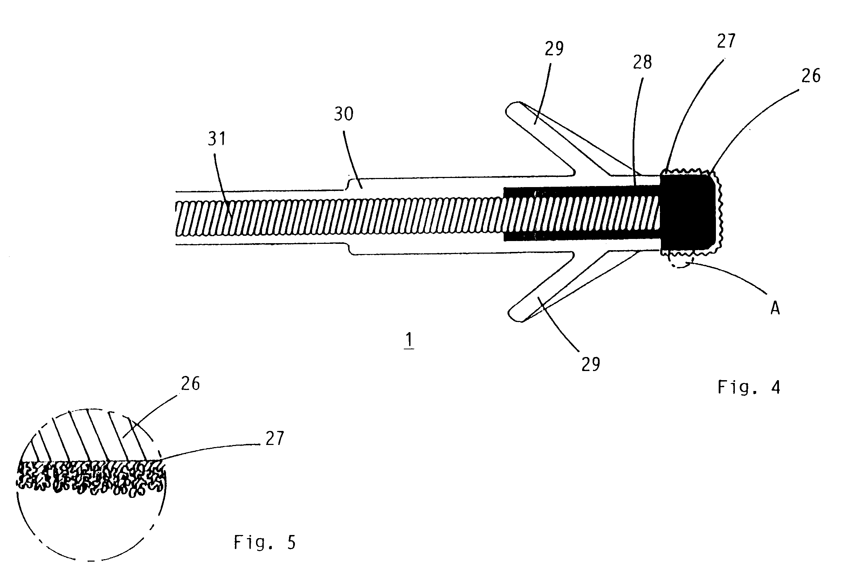

Bei der in Figur 4 in schematischer Seitansicht dargestellten Stimulationselektrode 1 handelt es sich um eine unipolare Noppenelektrode mit einem einen zylinderförmigen Grundkörper 26 aus Titan aufweisenden Kopf. Der zylinderförmige Grundkörper 26 weist erfindungsgemäß eine aus einem inerten Material Iridiumnitrid (IrN) bestehende Oberflächenbeschichtung 27 auf, die mittels Kathodenzerstäubung auf den zylinderförmigen Grundkörpers 26 der Titanelektrode aufgebracht ist. Die Elektrode weist eine gewendelte, elektrisch leitende Zuleitung 31 auf, die mit einer elektrisch isolierenden Ummantelung 30 aus Silikon versehen ist. In der Zeichnung ist diese Silikonummantelung transparent wiedergegeben. An die Silikonummantelung angeformt sind nach rückwärts gerichtete flexible Befestigungselemente 29, welche zur Verankerung der Elektrode im Herzen dienen, wobei die Oberfläche des Grundkörpers in Kontakt mit der inneren Herzoberfläche gehalten wird.

Der Grundkörper 26 ist mittels eines hohlzylindrischen Ansatzes 28 über die Zuleitung 31 geschoben und dort befestigt, wobei dieser Ansatz in der Zeichnung geschnitten dargestellt ist.

In Figur 5 ist ein Ausschnitt (Detail A in Figur 4) der aktiven Oberfläche vergrößerten wiedergegeben. Wie aus der Darstellung ersichtlich ist, wird durch die (unmaßstäblich vergrößerte) fraktale räumliche Geometrie der im mikroskopischen Bereich stengelartig gewachsenen Beschichtung 27 eine wesentliche Vergrößerung der aktiven Oberfläche erzielt. Die erzielte Oberflächenvergrößerung liegt im Bereich von mehr als 1000.

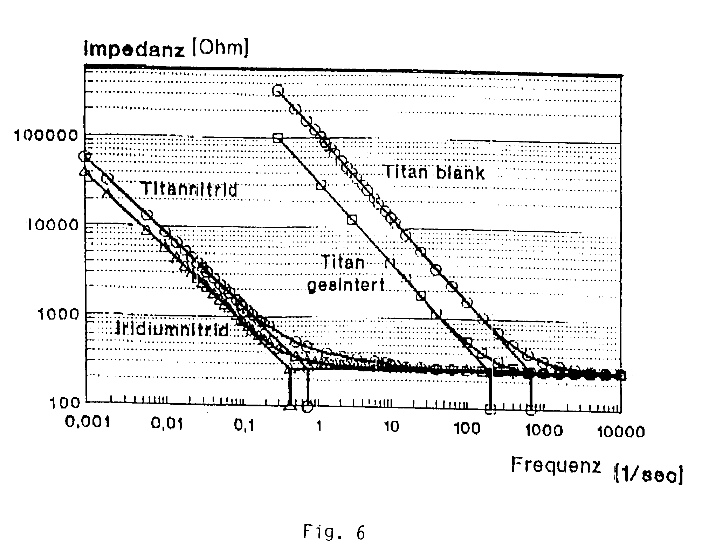

Aus Figur 6, die den Verlauf der Impedanzen von Stimulationselektroden mit unterschiedlichen Oberflächenbeschichtungen im Vergleich zeigt, ist ersichtlich, daß eine mit Iridiumnitrid beschichtete Elektrode insbesondere für den Empfang von aus dem Herzen aufnehmenden Signalen besonders wichtigen niederfrequenten Bereich im Vergleich zu den aus dem Stand der Technik bekannten Elektrodenoberflächenmaterialien Titan bzw. Titannitrid die niedrigste Phasengrenzimpendanz besitzt. Die ermittelten Unterschiede sind in ihren Auswirkungen deshalb besonders wesentlich, da die Amplitude des aufgenommenen Signals quadratisch mit dem Innenwiderstand der Signalquelle zusammenhängt.

In Figur 7 sind Meßergebnisse dargestellt, die das durch die Stimulation entstehende Nachpotential als Funktion der Autoshort-Zeit T in Abhängigkeit von der Konfiguration der Stimulationselektrode zeigen. Da das den Erfolg einer Myocard-Reizung anzeigende evozierte Potential in einem Zeitbereich von 50 bis 300 ms nach der Stimulation zu finden ist, kann dessen Detektion mit einer Titannitridbeschichteten Stimulationselektrode bei Autoshort-Zeiten von 50 ms störungsfrei erfolgen, wogegen bei unbeschichtete Platin-Elektroden das evozierte Potential durch Nachpotentiale in Größenordnung 10 mV "verdeckt" sind. Die Detektierung der evozierten Potentiale zu einem Zeitpunkt nach 50 ms wird auch dadurch erheblich erschwert und ist mit unbeschichteten Stimulationselektroden nicht möglich, da sich die Amlitude des evozierten Potentials nach Entstehen sehr schnell verringert und unter den Wert des verbleibenden Nachpotentials sinkt.

**Patentansprüche**

1. Herzschrittmachersystem, mit einer Schaltung (4, 5, 6) zur Erzeugung von Stimulationsimpulsen, einer Schaltung (11, 12, 13) zur Erfassung einer evozierten Herzaktion mittels eines durch eine im Kerzen verlegte Elektrode aufgenommenen elektrischen Signals und einer Schaltung (14, 15) zur Erzeugung eines Impulses zum Abbau der Restladung eines Ausgangskondensators (1) bzw. zur Beseitigung des Nachpotentials nach einem Stimulationsimpuls durch eine im Herzen angeordnete Stimulationselektrode,
**dadurch gekennzeichnet,**
daß die Stimulationselektrode eine poröse Oberflächenbeschichtung aus einem inerten Material, dessen aktive Oberfläche wesentlich größer ist als die Oberfläche der geometrischen Grundform der Elektrode, aufweist und
daß die Zeitdauer der Aktivierung der Schaltung zum Abbau der Restladung des Ausgangskondensators nach einem Stimulationsimpuls auf weniger als 70 ms begrenzt ist und
in Abhängigkeit von der Erfassung eines evozierten Impulses verändert wird.

2. Herzschrittmachersystem nach Anspruch 1, **dadurch gekennzeichnet,** daß Schaltungsmittel (16, 17) zur selbsttätigen Einstellung der Zeitdauer der Aktivierung der Schaltung (14, 15) zum Abbau der Restladung vorgesehen sind, welche während eines Zeitraums zur selbsttätigen Einstellung der Zeitdauer der Aktivierung der Schaltung zum Abbau der Restladung eine Stimulation mit heraufgesetzter Amplitude bewirken, während die Zeitdauer der Aktivierung der Schaltung zum Abbau der Restladung zwischen den einzelnen Stimulationsimpulsen sukzessiv variiert wird, bis mit der Schaltung (11, 12, 13) zur elektrischen Erfassung einer evozierten Herzaktion ein elektrisches Signal aufgenommen wird.

3. Herzschrittmachersystem nach Anspruch 2, **dadurch gekennzeichnet,** daß die Zeitdauer der Aktivierung der Schaltung zum Abbau der Restladung von einem Maximalwert ausgehend sukzessiv verringert wird.

4. Herzschrittmachersystem nach Anspruch 2, **dadurch gekennzeichnet,** daß die Schaltungsmittel zur selbsttätigen Einstellung der Zeitdauer der Aktivierung der Schaltung um Abbau der Restladung aufweisen:

- Schaltungsmittel (16) zur Variation der Zeitdauer der Aktivierung der Schaltung zum Abbau der Restladung,

- Schaltungsmittel (11, 12, 13) zur Detektierung und Speicherung der Erfassung einer evozierten Herzaktion (3, 25) in Zuordnung zur jeweiligen Zeitdauer der Aktivierung der Schaltung zum Abbau der Restladung,

- Schaltungsmittel (5, 7, 8, 9, 10) zur Heraufsetzung der Amplituden der Stimulationsimpulse (2, 24).

5. Herzschrittmachersystem nach einem der Ansprüche 2 bis 4, **gekennzeichnet durch** Schaltungsmittel (5, 7) zur Veränderung der Amplitude der Stimulationsimpulse, nachdem durch die Schaltungsmittel zur selbsttätigen Einstellung der Zeitdauer der Aktivierung der Schaltung zum Abbau der Restladung eine solche Zeitdauer ermittelt wurde, welche eine sichere Erfassung einer evozierten Herzaktion mittels eines durch die im Herzen verlegte Elektrode aufgenommenen Signals ermöglicht.

6. Herzschrittmachersystem nach Anspruch 5, **dadurch gekennzeichnet,** daß die Schaltungsmittel zur Veränderung der Amplitude der Stimulationsimpulse eine Regelschaltung aufweisen, welche, wenn die Schaltungsmittel zur selbsttätigen Einstellung der Zeitdauer der Aktivierung der Schaltung zum Abbau der Restladung nicht aktiviert sind, die Amplitude der Stimulationsimpulse um einen vorgegebenen Amplitudenbetrag vergrößern, wenn kein evoziertes Signal detektiert wird, und um einen vorgegebenen Amplitudenbetrag verkleinern, wenn ein evoziertes Signal detektiert wird.

7. Herzschrittmachersystem nach Anspruch 6, **dadurch gekennzeichnet,** daß bei der Detektion eines evozierten Signals (3, 25) die Amplitude des Stimulationsimpulses (2, 24) um einen ersten Amplitudenbetrag verringert wird, der kleiner ist als ein zweiter Amplitudenbetrag, um den die Stimulationsamplitude bei Ausbleiben eines evozierten Signals (3, 25) angehoben wird.

8. Herzschrittmachersystem nach Anspruch 6 oder 7, **dadurch gekennzeichnet,** daß bei jeder n-ten Detektion eines evozierten Signals (3, 25) die Amplitude des Stimulationsimpulses (2, 24) um einen vorgegebenen Amplitudenbetrag verringert wird.

9. Herzschrittmachersystem nach Anspruch 1, **dadurch gekennzeichnet,** daß zur Speicherung des Auftretens von evozierten Herzaktionen (3, 25) ein adressierbarer Speicher (12) vorgesehen ist.

10. Herzschrittmachersystem nach Anspruch 9, **dadurch gekennzeichnet,** daß die Zuordnung der evozierten und detektierten Potentiale (3, 25) zu Speicherplätzen des adressierbaren Speichers (12) in Abhängigkeit vom Ausgangssignale eines Rampengenerator (16) erfolgt.

11. Herzschrittmachersystem nach Anspruch 9, **dadurch gekennzeichnet,** daß dem adressierbaren Speicher (12) eine Auswerte-Einheit (13) nachgeordnet ist, die eine mittlere Dauer eines Impulses zum Abbau der Restladung bzw. zur Beseitigung des Nachpotentials ermittelt und die Schaltung zum Abbau der Restladung (15) in einem Betriebszustand "Dauerbetrieb" auf diese Impulsbreite fixiert.

12. Herzschrittmachersystem nach Anspruch 11, **dadurch gekennzeichnet,** daß ein Umschalter (14) am Eingang der Schaltung zum Abbau der Restladung zum Abbau der Restladung bzw. zur Beseitigung des Nachpotentials angeordnet ist und diesen in einem Betriebszustand "Abgleich" mit dem Rampengenerator (16) bzw. in einem Betriebszustand "Dauerbetrieb" mit der Auswerte-Einheit (13) verbindet.

13. Herzschrittmachersystem nach einem der Ansprüche 1 bis 12, **dadurch gekennzeichnet,** daß die aktive Oberfläche der Stimulationselektrode (1) durch eine, insbesondere fraktale, räumliche Geometrie um einen Faktor von mindestens tausend größer ist als die sich aus der geometrischen Grundform der Elektrode ergebende Oberfläche.

14. Herzschrittmachersystem nach einem der Ansprüche 1 bis 13, **dadurch gekennzeichnet,** daß als inertes Material ein Nitrid, Carbid oder Carbonnitrid oder aber ein reines Element bzw. eine Legierung aus der Gruppe Gold, Silber, Titan, Iridium, Platin oder Kohlenstoff vorgesehen ist.

15. Herzschrittmachersystem nach einem der Ansprüche 1 bis 14, **dadurch gekennzeichnet,** daß die Oberflächenbeschichtung mittels Dünnschichttechnologie auf die Elektrode (1) aufgebracht ist.

16. Herzschrittmachersystem nach Anspruch 15, **dadurch gekennzeichnet,** daß die Oberflächenbeschichtung mittels reaktiver Kathodenzerstäubung oder Ionenplattierung auf die Elektrode (1) aufgebracht ist.

17. Herzschrittmachersystem nach einem der Ansprüche 1 bis 16, **dadurch gekennzeichnet,** daß der Grundkörper der Elektrode (1) aus Titan besteht.

## Claims

1. Heart pacemaker system including a circuit (4, 5, 6) for producing stimulating pulses, a circuit (11, 12, 13) for detecting an evoked action of the heart by means of an electrical signal received by an electrode placed in the heart, and a circuit (14, 15) for producing a pulse for reducing the residual charge on an output capacitor (1) or for removing the afterpotential following a stimulating pulse through a stimulating electrode arranged in the heart, characterised in

   that the stimulating electrode comprises a porous surface layer consisting of an inert material whose active surface is substantially greater than the surface of the basic geometrical shape of the electrode and

   that the period of time, for which the circuit for reducing the residual charge on the output capacitor is activated after a stimulating pulse, is limited to less than 70 msecs and is altered in dependence on the detection of an evoked pulse.

2. Heart pacemaker system in accordance with Claim 1, characterised in that, there are provided circuit means (16, 17) for automatically setting the period of time for which the circuit (14, 15) for reducing the residual charge is activated, which said means cause a stimulating action having a raised amplitude during a time interval for the automatic setting of the period of time for which the circuit for reducing the residual charge is activated, whilst the period of time for which the circuit for reducing the residual charge is activated is successively varied between the individual stimulating pulses until an electrical signal is received by the circuit (11, 12, 13) for electrically detecting an evoked action of the heart.

3. Heart pacemaker system in accordance with Claim 2, characterised in that, the period of time, for which the circuit for reducing the residual charge is activated, is successively reduced starting from a maximum value.

4. Heart pacemaker system in accordance with Claim 2, characterised in that, the circuit means for automatically setting the period of time for which the circuit for reducing the residual charge is activated comprise:

   - circuit means (16) for varying the period of time for which the circuit for reducing the residual charge is activated,
   - circuit means (11, 12, 13) for detecting and storing the detection of an evoked action of the heart (3, 25) in relation to the prevailing period of time for which the circuit for reducing the residual charge is activated,
- circuit means (5, 7, 8, 9, 10) for raising the amplitude of the stimulating pulses (2, 24).

5. Heart pacemaker system in accordance with any of the Claims 2 to 4, characterised by circuit means (5, 7) for altering the amplitude of the stimulating pulses after a period of time which is such as to enable positive detection of an evoked action of the heart by means of a signal received by an electrode placed in the heart, said period of time being determined by the circuit means for automatically setting the period of time for which the circuit for reducing the residual charge is activated.

6. Heart pacemaker system in accordance with Claim 5, characterised in that, the circuit means for altering the amplitude of the stimulating pulses comprise a regulating circuit which, when the circuit means for automatically setting the period of time for which the circuit for reducing the residual charge is activated are not activated, increases the amplitude of the stimulating pulses by a predetermined amount when an evoked signal has not been detected and reduces the amplitude by a predetermined amount when an evoked signal is detected.

7. Heart pacemaker system in accordance with Claim 6, characterised in that, in the case of the detection of an evoked signal (3, 25), the amplitude of the stimulating pulse (2, 24) is reduced by a first amount which is smaller than a second amount, by which the stimulating amplitude is raised in the case of absence of an evoked signal (3, 25).

8. Heart pacemaker system in accordance with Claim 6 or 7, characterised in that, the amplitude of the stimulating pulse (2, 24) is reduced by a predetermined amount for each $n^{th}$ detection of an evoked signal (3, 25).

9. Heart pacemaker system in accordance with Claim 1, characterised in that, an addressable memory (12) is provided for storing the appearance of an evoked action of the heart (3, 25).

10. Heart pacemaker system in accordance with Claim 9, characterised in that, the allocation of the evoked and detected potentials (3, 25) to storage locations in the addressable memory (12) is effected in dependence on the output signals from a ramp generator (16).

11. Heart pacemaker system in accordance with Claim 9, characterised in that, under the addressable memory (12) there is a subordinate evaluating unit (13) which establishes an average value for the duration of a pulse for reducing the residual charge or for removing the after-potential and which sets the circuit (15) for reducing the residual charge into an operational mode "continuous operation" for this pulse width.

12. Heart pacemaker system in accordance with Claim 11, characterised in that, a switch (14) is arranged at the input of the circuit for reducing the residual charge [sic] for reducing the residual charge or for removing the after-potential and said switch connects said input to the ramp generator (16) in an operational mode "compare" or connects said input to the evaluating unit (13) in an operational mode "continuous operation".

13. Heart pacemaker system in accordance with any of the Claims 1 to 12, characterised in that, the active surface of the stimulating electrode (1) is greater by a factor of at least one thousand, due especially to its fractal three dimensional geometry, than the surface resulting from the basic geometrical shape of the electrode.

14. Heart pacemaker system in accordance with any of the Claims 1 to 13, characterised in that, a nitride, carbide, carbonitride or even a pure element or an alloy selected from the group gold, silver, titanium, iridium, platinum or carbon is used as the inert material.

15. Heart pacemaker system in accordance with any of the Claims 1 to 14, characterised in that, the surface layer is deposited on the electrode (1) using thin-film techniques.

16. Heart pacemaker system in accordance with Claim 15, characterised in that, the surface layer is deposited on the electrode (1) by means of a reactive cathodic sputtering process or an ionic plating process.

17. Heart pacemaker system in accordance with any of the Claims 1 to 16, characterised in that, the body of the electrode (1) consists of titanium.

**Revendications**

1. Stimulateur cardiaque, équipé d'un circuit (4, 5, 6) de génération d'impulsions de stimulation, d'un circuit (11, 12, 13) de détection d'une action cardiaque provoquée au moyen d'un signal électrique reçu par une électrode placée dans le coeur, et d'un circuit (14, 15) de génération d'une impulsion de suppression de la charge résiduelle d'un condensateur de sortie (1) ou en vue de la suppression du potentiel résiduel après une impulsion de stimulation par une électrode de stimulation placée dans le coeur, caractérisé en ce que l'électrode de stimulation présente un revêtement superficiel poreux en un matériau inerte, dont la surface active est sensiblement supérieure à la surface de la forme de base géométrique de l'électrode, et en ce que la durée de l'activation du circuit de suppression de charge résiduelle du condensateur de sortie après une impulsion de stimulation est limitée à moins de 70 ms, et est modifiée en fonction de la détection d'une impulsion provoquée.

2. Stimulateur cardiaque selon la revendication 1, caractérisé en ce que des moyens de circuit (16, 17) sont prévus en vue du réglage automatique de la durée de l'activation du circuit (14, 15) en vue de la suppression de la charge résiduelle, qui provoquent pendant une durée du réglage automatique de la durée d'activation du circuit de suppression de la charge résiduelle une stimulation d'amplitude accrue, pendant la durée de l'activation du circuit de suppression de la charge résiduelle entre les différentes impulsions de stimulation est modifiée successivement, jusqu'à ce que, avec le circuit (11, 12, 13) de détection électrique d'une réaction cardiaque provoquée, un signal électrique soit reçu.

3. Stimulateur cardiaque selon la revendication 2, caractérisé en ce que la durée de l'activation du circuit de suppression de la charge résiduelle est réduite successivement à partir d'une valeur maximale.

4. Stimulateur cardiaque selon la revendication 2, caractérisé en ce que les moyens de circuit de réglage automatique de la durée de l'activation du circuit de suppression de la charge résiduelle comportent :

   Des moyens de circuit (16) en vue de la variation de la durée de l'activation du circuit de suppression de la charge résiduelle,
   Des moyens de circuit (11, 12, 13) en vue de la détection et de la mémorisation de la détection d'une action cardiaque provoquée (3, 25) en association avec la durée respective de l'activation du circuit de suppression de la charge résiduelle,
   Des moyens de circuit (5, 7, 8, 9, 10) en vue de l'accroissement des amplitudes des impulsions de stimulation (2, 24).

5. Stimulateur cardiaque selon une des revendications 2 à 4, caractérisé par des moyens de circuit (5, 7) en vue de la variation de l'amplitude des impulsions de stimulation, après que, grâce aux moyens de circuit de réglage automatique de la durée de l'activation du circuit de suppression de la charge résiduelle, une telle durée a été détectée, qui permet une détection sûre d'une action cardiaque provoquée au moyen d'un signal reçu par l'électrode placée dans le coeur.

6. Stimulateur cardiaque selon la revendication 5, caractérisé en ce que les moyens de circuit de variation de l'amplitude des impulsions de stimulation présentent un circuit de régulation, qui, lorsque les moyens de circuit de réglage automatique de la durée de l'activation du circuit de suppression de la charge résiduelle ne sont pas activés, augmentent l'amplitude des impulsions de stimulation d'un degré d'amplitude prédéterminé, lorsqu'aucun signal provoqué n'est détecté, et réduisent d'un degré d'amplitude prédéterminé, lorsqu'un signal provoqué est détecté.

7. Stimulateur cardiaque selon la revendication 6, caractérisé en ce que, lors de la détection d'un signal provoqué (3, 25), l'amplitude de l'impulsion de stimulation (2, 24) est réduite d'un premier degré d'amplitude, qui est inférieur à un second degré d'amplitude dont l'amplitude de stimulation est accrue lors de l'absence du signal provoqué (3, 25).

8. Stimulateur cardiaque selon la revendication 6 ou 7, caractérisé en ce que, lors de chaque n-ième détection d'un signal provoqué (3, 35), l'amplitude de l'impulsion de stimulation (2, 24) est réduite d'un degré d'amplitude prédéterminé.

9. Stimulateur cardiaque selon la revendication 1, caractérisé en ce qu'en vue de la mémorisation de l'apparition d'actions cardiaques provoquées (3, 25) est prévue une mémoire adressable (12).

10. Stimulateur cardiaque selon la revendication 9, caractérisé en ce que l'association des potentiels provoqués et détectés (3, 25) s'effectue en des emplacements de mémoire de la mémoire adressables (12) en fonction de signaux de sortie d'un générateur de rampe (16).

11. Stimulateur cardiaque selon la revendication 9, caractérisé en ce qu'en aval de la mémoire adressable (12) est disposée une unité d'évaluation (13), qui détecte une durée moyenne d'une impulsion de suppression de la charge résiduelle ou en vue de la suppression du potentiel résiduel et fixent le circuit de suppression de la charge résiduelle (15) dans un état de fonctionnement "fonctionnement permanent" sur cette largeur d'impulsion.

12. Stimulateur cardiaque selon la revendication 11, caractérisé en ce qu'un commutateur (14) est disposé à l'entrée du circuit de suppression de la charge résiduelle et relie celui-ci dans un état de fonctionnement "réglage" avec le générateur de rampe (16) ou dans un état de fonctionnement "fonctionnement permanent" avec l'unité d'évaluation (13).

13. Stimulateur cardiaque selon l'une des revendications 1 à 12, caractérisé en ce que la surface active de l'électrode de stimulation (1) est supérieure par une géométrie spatiale, en particulier fractale, d'un facteur d'au moins 1000 que la surface résultant de la forme de base géométrique de l'électrode.

14. Stimulateur cardiaque selon une des revendications 1 à 13, caractérisé en ce qu'en tant que matériau inerte est prévu un nitrure, un carbure ou un nitrure de carbone ou un élément pur ou un alliage du groupe or, argent, titane, iridium, platine ou carbone.

15. Stimulateur cardiaque selon une des revendications 1 à 14, caractérisé en ce que le revêtement superficiel est déposé sur l'électrode (1) par technologie des couches minces.

16. Stimulateur cardiaque selon la revendication 15, caractérisé en ce que le revêtement superficiel est déposé par pulvérisation cathodique réactive ou placage ionique sur l'électrode (1).

17. Stimulateur cardiaque selon une des revendications 1 à 16, caractérisé en ce que l'élément fondamental de l'électrode (1) est du titane.

Fig. 1

Fig. 2

EP 0 660 734 B1

Fig. 3

Fig. 4

Fig. 5

Fig. 6

Fig. 7

EP 0 660 734 B1